# EUROPÄISCHE PATENTSCHRIFT

(11) **EP 0 537 620 B1**
(45) Veröffentlichungstag und Bekanntmachung des Hinweises auf die Patenterteilung: **07.01.1998**
(21) Anmeldenummer: 92117159.1
(22) Anmeldetag: 08.10.1992
(51) Int. Cl.: C07D 239/34, C09K 19/34, C07D 213/65, G02F 1/13

(54) **Stickstoff-Heterocyclen**
Nitrogen-containing heterorings
Hétérocycles azotés

(30) Priorität: 16.10.1991 CH 3034/91; 28.08.1992 CH 2679/92
(43) Veröffentlichungstag der Anmeldung: 21.04.1993
(73) Patentinhaber: Rolic AG, 6301 Zug (CH)
(72) Erfinder: Buchecker, Richard, CH-8008 Zürich (CH); Kelly, Stephen, CH-4313 Möhlin (CH); Schadt, Martin, CH-4411 Seltisberg (CH)
(74) Vertreter: Eder, Carl E.

(56) Entgegenhaltungen:
- EP-A- 0 435 632
- DE-A- 3 939 982

## Beschreibung

Die vorliegende Erfindung betrifft Stickstoff-Heterocyclen, flüssigkristalline Gemische, die solche Verbindungen enthalten, sowie die Verwendung für elektro-optische Zwecke.

Flüssige Kristalle werden vor allem als Dielektrika in Anzeigevorrichtungen eingesetzt, da die optischen Eigenschaften solcher Substanzen durch eine angelegte Spannung beeinflusst werden können. Elektro-optische Vorrichtungen auf der Basis von Flüssigkristallen sind dem Fachmann bestens bekannt und können auf verschiedenen Effekten beruhen. Beispiele solcher Vorrichtungen sind Zellen mit dynamischer Streuung, DAP-Zellen (Deformation aufgerichteter Phasen), SSF-Zellen (surface stabilized ferroelectric), DHF-Zellen (deformed helix ferroelectric) oder SBF-Zellen (short-pitch bistable ferroelectric).

Die Flüssigkristallmaterialien müssen eine gute chemische und thermische Stabilität und eine gute Stabilität gegenüber elektrischen Feldern und elektromagnetischer Strahlung besitzen. Ferner sollten die Flüssigkristallmaterialien niedere Viskosität aufweisen und in den Zellen kurze Ansprechzeiten und einen hohen Kontrast ergeben. Weiterhin sollten sie bei üblichen Betriebstemperaturen von etwa -30°C bis etwa +80°C, insbesondere von etwa -20°C bis etwa +60°C eine geeignete Mesophase besitzen, beispielsweise für die oben genannten Zellen eine breite smektische Mesophase.

Da Flüssigkristalle in der Begel als Mischungen mehrerer Komponenten zur Anwendung gelangen, ist es zudem wichtig, dass die Komponenten untereinander gut mischbar sind. **In EP-A-0 435 632 sind flüssigkristalline Pyrimidinderivate offenbart, welche für obengenannte Zwecke verwendet werden können.**

Gegenstand der vorliegenden Erfindung sind die Verbindungen der allgemeinen Formel 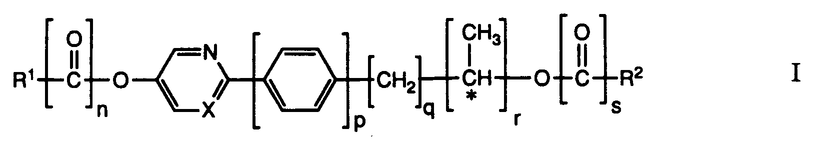 worin
- R¹: Alkyl oder Alkenyl mit 5 bis 12 Kohlenstoffatomen bedeutet;
- R²: Alkyl oder Alkenyl mit 1 bis 9 bzw. 2 bis 9 Kohlenstoffatomen bedeutet; mit der Massgabe, dass mindestens eine der Gruppen R¹ oder R² Alkenyl bedeutet;
- X: eine CH-Gruppe oder Stickstoff bedeutet;
- n: entweder 0 oder 1 ist;
- q: eine ganze Zahl von 2 bis 6 ist;
- r: entweder 0 oder 1 ist, mit der Massgabe, dass Σ q+r mindestens 3 ist;
- s: entweder 0 oder 1 ist;
- p: 1 oder 2 bedeutet, mit der Massgabe, dass wenn p = 2 ist, n für die Zahl 0 steht.

Der Ausdruck "Alkyl" umfasst im Rahmen der vorliegenden Erfindung geradkettige Reste R¹ mit 5 bis 12 Kohlenstoffatomen bzw. R² mit 1 bis 9 Kohlenstoffatomen wie Methyl, Aethyl, Propyl, Butyl, Pentyl, Hexyl, Heptyl, Octyl, Nonyl, Decyl, Undecyl und Dodecyl.

Der Ausdruck "Alkenyl" umfasst geradkettige Reste R¹ mit 5 bis 12 bzw. R² mit 2 bis 9 Kohlenstoffatomen. Solche Reste sind beispielsweise Allyl, 2E-Butenyl, 2E-Pentenyl, 2E-Hexenyl, 2E-Heptenyl, 2E-Octenyl, 2E-Nonenyl, 2E-Decenyl, 2E-Undecenyl, 2E-Dodecenyl, 3-Butenyl, 3Z-Pentenyl, 3Z-Hexenyl, 3Z-Heptenyl, 3Z-Octenyl, 3Z-Nonenyl, 3Z-Decenyl, 3Z-Undecenyl, 3Z-Dodecenyl, 4-Pentenyl, 4E-Hexenyl, 4E-Heptenyl, 4E-Octenyl, 4E-Nonenyl, 4E-Decenyl, 4E-Undecenyl, 4E-Dodecenyl, 5-Hexenyl, 5Z-Heptenyl, 5Z-Octenyl, 5Z-Nonenyl, 5Z-Decenyl, 5Z-Undecenyl, 5Z-Dodecenyl, 6-Heptenyl, 6E-Octenyl, 6E-Nonenyl, 6E-Decenyl, 6E-Undecenyl, 6E-Dodecenyl, 7-0ctenyl, 7Z-Nonenyl, 7Z-Decenyl, 7Z-Undecenyl, 7Z-Dodecenyl, 8-Nonenyl, 8E-Decenyl, 8E-Undecenyl, 8E-Dodecenyl, 9-Decenyl, 9Z-Undecenyl, 9Z-Dodecenyl, 10-Undecenyl, 10E-Dodecenyl, 11-Dodecenyl und dergleichen.

Verbindungen der allgemeinen Formel I, worin r = 1 ist, eignen sich in hervorragender Weise als Dotierstoffe für ferro-elektrische Flüssigkristall-Mischungen, da sie zum Teil selbst eine smektische C Phase aufweisen.

Besonders bevorzugte Verbindungen sind achirale Verbindungen der allgemeinen Formel 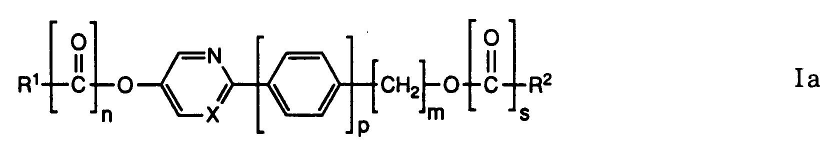 worin R¹, R², X, n, p und s die obengenannten Bedeutungen haben und m eine ganze Zahl von 3 bis 6 ist.

Besonders bevorzugte Verbindungen sind Verbindungen der Formel I und Ia, worin R¹ Alkenyl mit 5 bis 10 Kohlenstoffatomen bezeichnet, R² Alkyl mit 1 bis 7 Kohlenstoffatomen bedeutet und X Stickstoff bedeutet.

Ganz besonders bevorzugt sind demnach Verbindungen der Formel 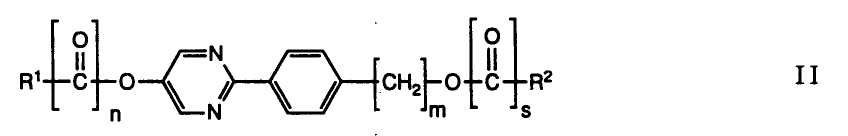 worin R¹ Alkenyl mit 8 bis 10 Kohlenstoffatomen bedeutet; R² Alkyl mit 1 bis 5 Kohlenstoffatomen bezeichnet; und n, m und s obige Bedeutung haben.

Es wurde überraschenderweise gefunden, dass die erfindungsgemässen Verbindungen ausserordentlich günstige Mesophasen für ferro-elektrische Anwendungen aufweisen. Die erfindungsgemässen Verbindungen weisen breite smektisch C Mesophasen mit erstaunlich niedriger Viskosität auf, was zu sehr kurzen Schaltzeiten führt. Der Schmelzpunkt der erfindungsgemässen Verbindungen ist oft sehr niedrig. Dies bewirkt eine signifikante Absenkung des Schmelzpunktes von Mischungen, welche solche Verbindungen enthalten, und führt somit zu vergleichsweise breiten smektischen Mesophasen.

Die erfindungsgemässen Verbindungen der Formel I, worin n = 1 ist, können in an sich bekannter Weise aus 5-Hydroxypyrimidinen bzw. 5-Hydroxypyrimidinen und einer Säure hergestellt werden. Die Umsetzung kann beispielsweise in Gegenwart von N,N'-Dicyclohexylcarbodiimid und 4-(Dimethylamino)pyridin in Dichlormethan oder einem anderen geeigneten Lösungsmittel wie z.B. Chloroform erfolgen.

Die erfindungsgemässen Verbindungen der Formel I, worin n = 0 ist, können in an sich bekannter Weise ebenfalls aus den entsprechenden 5-Hydroxypyrimidinen bzw. 5-Hydroxypyridinen und 1-Hydroxyalkenen hergestellt werden. So kann 5-Hydroxy-2-(p-substituiertes)-phenyl-pyrimidin beispielsweise unter Mitsunobu-Bedingungen d.h. in Gegenwart von Azodicarbonsäure-diäthylester und Triphenylphosphin in Tetrahydrofuran mit einem Hydroxyalken umgesetzt werden.

Die Verbindungen der Formel I können in Form von Gemischen untereinander und/oder mit anderen Flüssigkristallkomponenten verwendet werden. Sie werden vorzugsweise in Gemischen mit anderen Flüssigkristallkomponenten verwendet.

Die erfindungsgemässen flüssigkristallinen Gemische enthalten mindestens 2 Komponenten, wovon mindestens eine Komponente eine Verbindung der Formel I ist. Eine zweite und gegebenenfalls weitere Komponenten können weitere Verbindungen der Formel I oder auch andere flüssigkristalline Verbindungen sein. Es muss jedoch jeweils mindestens eine chirale Komponente im Gemisch enthalten sein. Diese chirale Verbindung kann entweder eine chirale Verbindung der Formel I sein oder ein bekannter chiraler Dotierstoff.

Derartige Flüssigkristallkomponenten sind vorzugsweise achirale Verbindungen der Formeln 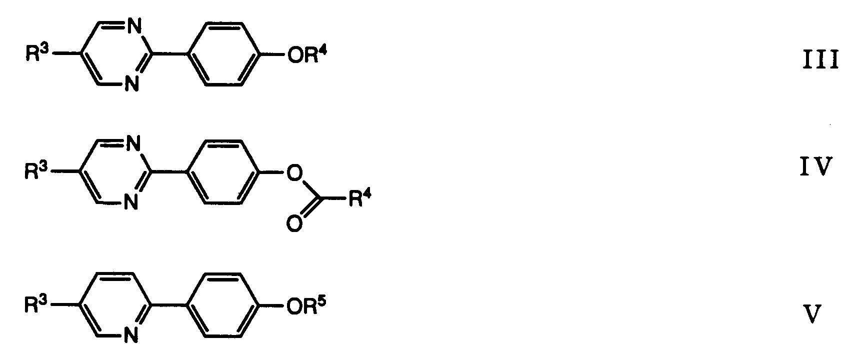 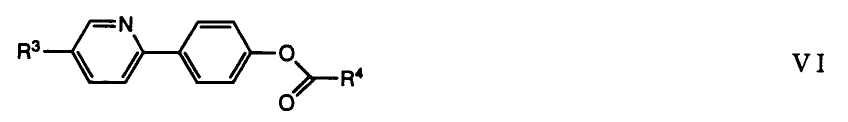 bzw. chirale Dotierstoffe der allgemeinen Formeln 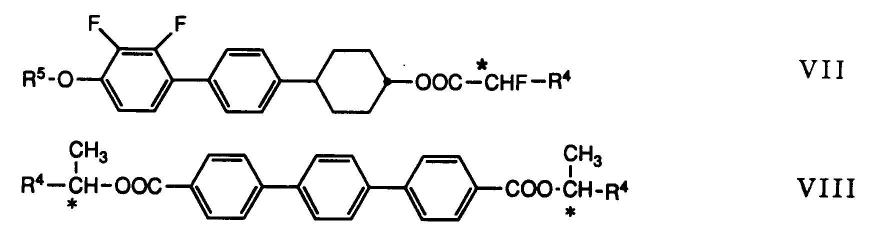 worin R³ Alkyl oder Alkoxy bedeutet; R⁴ Alkyl bedeutet; und R⁵ Alkyl oder Alkenyl bedeutet.

Der Ausdruck"Alkyl" im Zusammenhang mit den Verbindungen der Formeln III bis VIII umfasst unverzweigte und verzweigte Alkylgruppen mit 1 bis 15 Kohlenstoffatomen, vorzugsweise unverzweigte Alkylgruppen mit 1-12 Kohlenstoffatomen wie Methyl, Aethyl, Propyl, Butyl, Pentyl, Hexyl, Heptyl, Octyl, Nonyl, Decyl, Undecyl, Dodecyl und dergleichen.

Der Ausdruck "Alkoxy" umfasst Aethergruppen in denen der Alkylrest wie vorhergehend definiert ist.

Der Ausdruck "Alkenyl" umfasst Alkenylgruppen mit 2 bis 15 Kohlenstoffatomen, wie 2E-Alkenyl-, 3Z-Alkenyl-, 4-Alkenyl- und Alkenyl-Reste mit endständiger Doppelbindung, vorzugsweise Alkenylgruppen mit 2 bis 9 Kohlenstoffatomen.

Die Ausdrücke "2E-Alkenyl", "3Z-Alkenyl" und 4E-Alkenyl" umfassen unverzweigte Alkenylgruppen mit 3 bis 15, 4 bis 15 bzw. 5 bis 15 Kohlenstoffatomen, in welchen die Doppelbindung in 2, 3 bzw. 4 Stellung steht, wobei E und Z die Konfiguration der Doppelbindung bezeichnet. Solche Gruppen sind beispielsweise Allyl, 2E-Butenyl, 2E-Pentenyl, 2E-Hexenyl, 2E-Heptenyl, 2E-Octenyl, 2E-Nonenyl, 2E-Decenyl, 3-Butenyl, 3Z-Pentenyl, 3Z-Hexenyl, 3Z-Heptenyl, 3Z-Octenyl, 3Z-Nonenyl, 3Z-Decenyl, 4-Pentenyl, 4E-Hexenyl, 4E-Heptenyl, 4E-Octenyl, 4E-Nonenyl, 4E-Decenyl und dergleichen. Der Ausdruck "Alkenyl" mit endständiger Doppelbindung umfasst unverzweigte Alkenyle mit 2 bis 15 Kohlenstoffatomen wie beispielsweise Allyl, 3-Butenyl, 4-Pentenyl, 5-Hexenyl, 6-Heptenyl, 7-Octenyl, 8-Nonenyl, 9-Decenyl und dergleichen.

Aufgrund der guten Löslichkeit der Verbindungen der Formel I und aufgrund ihrer guten Mischbarkeit untereinander, kann der Anteil an Verbindungen der Formel I in den erfindungsgemässen Gemischen relativ hoch sein und bis etwa 85 Gew.-% betragen. Bevorzugt wird jedoch im allgemeinen ein Anteil von Verbindungen der Formel I von etwa 1-50 Gew.-%, insbesondere von etwa 5-35 Gew.-%.

Die Herstellung der flüssigkristallinen Gemische und der elektrooptischen Vorrichtungen kann in an sich bekannter Weise erfolgen.

Die Erfindung wird durch die folgenden Beispiele weiter veranschaulicht. In den Beispielen bedeutet C eine kristalline, N eine nematische, S eine smektische und I die isotrope Phase.

### Beispiel 1

Zu einer Lösung von 1,0 g 2-[4-(5-[Propyloxy]-1-pentyl)phenyl]-5-hydroxypyrimidin, 0,4 g (Z)-3-Octensäure und 0,05 g 4-(Dimethylamino)pyridin in 30 ml Dichlormethan wurde unter Rühren innert 5 Minuten 0,8 g N,N'-Dicyclohexylcarbodiimid gegeben. Das Reaktionsgemisch wurde über Nacht weiter gerührt, dann filtriert und das Filtrat mit gesättigter Natriumbicarbonat-Lösung und mit Wasser gewaschen und eingeengt. Chromatographie des Rückstandes an Kieselgel mit Hexan/Aethylacetat (Vol. 4:1) und zweimaliges Umkristallisieren der gemäss Dünnschichtchromatographie reinen Fraktionen ergab 1,0 g 2-[4-(5-[Propyloxy]-1-pentyl)phenyl]-5-{[(Z)-3-octenoyl]oxy}pyrimidin.

Das als Ausgangsmaterial verwendete 2-[4-(5-[Propyloxy]-1-pentyl)phenyl]-5-hydroxypyrimidin wurde wie folgt hergestellt:
a) Eine Lösung von 30 g 4-Brom-benzonitril, 23 ml Aethylalkohol und 200 ml Toluol wurde bei 0°C vorgelegt und während 6 Stunden Chlorwasserstoff eingeleitet. Das Reaktionsgemisch wurde noch 48 Stunden bei Raumtemperatur weitergerührt, dann eingeengt und unter Vakuum getrocknet. Dies ergab 39 g Aethyl-4-brombenzimidat-hydrochlorid mit Smp. 250°C (Zersetzung).
b) Ein Gemisch von 39 g Aethyl-4-brombenzimidat-hydrochlorid und 100 mi Aethylalkohol wurde bei Raumtemperatur und unter Stickstoffbegasung vorgelegt, dann mit 82,5 ml gesättigter äthanolischer Ammoniak-Lösung versetzt und über Nacht gerührt. Das Reaktionsgemisch wurde eingeengt, unter Vakuum getrocknet, in Diäthyläther suspendiert, 2 Stunden bei Raumtemperatur gerührt, auf 0°C abgekühlt und abgenutscht. Die weissen Kristalle wurden mit Diäthyläther gewaschen und dann unter Vakuum getrocknet. Dies ergab 35,7 g 4-Brombenzamidin-hydrochlorid mit Smp. 265°C.
c) Eine Natriummethylat-Lösung frisch hergestellt aus 50 ml Methanol und 2,6 g Natrium wurde unter Stickstoffbegasung mit 15 g 4-Brombenzamidin-hydrochlorid und 13,5 g 1,3-bis(Dimethylamino)-2-äthoxytrimethinium-perchlorat (Cell. Czech. Chem. Commun. 38, 1168, 1973) in 80 ml Methanol versetzt. Das Reaktionsgemisch wurde 5 Stunden unter leichtem Rückfluss erwärmt, dann auf 100 ml Wasser gegossen, mit konzentrierter Salzsäure neutralisiert und dann dreimal mit je 100 ml Diäthyläther extrahiert. Die vereinigten organischen Phasen wurden mit 100 ml konzentrierter Kochsalz-Lösung, 100 ml konzentrierter Kaliumcarbonat-Lösung und dann nochmals mit 100 ml konzentrierter Kochsalz-Lösung gewaschen, über Magnesiumsulfat getrocknet, filtriert und anschliessend eingeengt. Der Rückstand wurde an Kieselgel mit Hexan/Aethylacetat (Vol. 9:1) chromatographiert und dann aus Aethylalkohol umkristallisiert. Dies ergab 14,7 g reines 2-(4-Bromphenyl)-5-(äthoxy)pyrimidin mit 135°C.
d) Ein Gemisch von 14,7 g 2-(4-Bromphenyl)-5-(äthoxy)pyrimidin, 5,7 g 4-Penten-1-ol, 10 g Natriumbicarbonat und 20 ml N-Methylpyrrolidon wurde mit 0,3 g Triphenylphosphin und 0,12 g Palladiumacetat versetzt. Das Reaktionsgemisch wurde 2 Stunden bei 120°C und unter einer Stickstoffatmosphäre erwärmt, dann auf 100 ml Wasser gegossen und dreimal mit je 50 ml Diäthyläther extrahiert. Die vereinigten organischen Phasen wurden zweimal mit je 100 ml konzentrierter Kochsalz-Lösung gewaschen, über Magnesiumsulfat getrocknet, filtriert und anschliessend eingeengt. Chromatographie des Rückstandes an Kieselgel mit Hexan/Aethylacetat (Vol. 8:2) und Umkristallisation aus Aethylalkohol ergab 12 g 2-[4-(5-Hydroxy-1-pentenyl)phenyl]-5-(äthoxy)pyrimidin.
e) Ein Gemisch von 12 g 2-[4-(5-Hydroxy-1-pentenyl)phenyl]-5-(äthoxy)pyrimidin, 2 g Palladium auf Aktivkohle (10 w/w %) und 150 ml Aethylacetat wurde bei Normaldruck und Raumtemperatur bis zur Aufnahme eines Moläquivalents von Wasserstoff hydriert. Das anorganische Material wurde abfiltriert und das Filtrat eingeengt. Der Rückstand wurde an Kieselgel mit Hexan/Aethylacetat (Vol. 8:2) chromatographisch gereinigt. Umkristallisation aus Aethylalkohol ergab 8,5 g reines 2-[4-(5-Hydroxy-1-pentyl)phenyl]-5-(äthoxy)pyrimidin.
f) Ein Gemisch von 1,2 g Natriumhyrid (60-65 wt%) und 150 ml Tetrahydrofuran wurde unter Stickstoffbegasung mit 8,5 g 2-[4-(5-Hydroxy-1-pentyl)phenyl]-5-(äthoxy)pyrimidin versetzt, 5 Stunden gerührt, mit 4 g Propylbromid versetzt und anschliessend über Nacht bei Raumtemperatur gerührt. Das Reaktionsgemisch wurde mit 500 mi Wasser versetzt und dreimal mit je 50 ml Diäthyläther extrahiert. Die vereinigten organischen Phasen wurden zweimal mit je 500 ml konzentrierter Kochsalz-Lösung gewaschen, über Magnesiumsulfat getrocknet, filtriert und anschliessend eingeengt. Chromatographie des Rückstandes an Kieselgel mit Hexan/Aethylacetat (Vol. 9:1) und Umkristallisation aus Aethylalkohol ergab 7,5 g reines 2-[4-(5-[Propyloxy]-1-pentyl)phenyl]-5-(äthoxy)pyrimidin.
g) Ein Gemisch von 7,5 g 2-[4-(5-[Propyloxy]-1-pentyl)phenyl]-5-(äthoxy)pyrimidin, 10 g Natriumhydroxid und 120 ml Diäthylenglykol wurde 8 Stunden bei 180°C erwärmt. Das Reaktionsgemisch wurde auf 100 ml Wasser gegossen, mit konzentrierter Salzsäure angesäuert, dann dreimal mit je 50 ml Diäthyläther extrahiert. Die vereinigten organischen Phasen wurden zweimal mit 100 ml konzentrierter Kochsalz-Lösung gewaschen, über Magnesiumsulfat getrocknet, filtriert und anschliessend eingeengt. Chromatographie des Rückstandes an Kieselgel mit Hexan/Aethylacetat (Vol. 7:3) und Umkristallisation aus Aethylalkohol ergab 5,2 g 2-[4-(5-[Propyloxy]-1-pentyl)phenyl]-5-hydroxypyrimidin.

In analoger Weise können folgende Verbindungen hergestellt werden:
2-[4-(5-[Propyloxy]-1-pentyl)phenyl]-5-([(E)-2-octenoyl]oxy)pyrimidin, Smp. (C-I) 68°C, (N-I) (64°C);
2-[4-(5-[Propyloxy]-1-pentyl)phenyl]-5-([(E)-2-nonenoyl]oxy)pyrimidin,;
2-[4-(5-[Propyloxy]-1-pentyl)phenyl]-5-([E)-2-decenoyl]oxy)pyrimidin, Smp. (C-N) 63°C, S_{C}-N(61°C), Klp. (N-I) 69°C;
2-[4-(5-[Propyloxy]-1-pentyl)phenyl]-5-([E)-2-dodecenoyl]oxy)pyrimidin, Smp. (C-S_{C}) 63°C, Klp. (S_{C}-I) 73°C;
2-[4-(5-[Propyloxy]-1-pentyl)phenyl]-5-([(Z)-3-nonenoyl]oxy)pyrimidin,
2-[4-(5-[Propyloxy]-1-pentyl)phenyl]-5-([(Z)-3-decenoyl]oxy)pyrimidin,
2-[4-(5-[Propyloxy]-1-pentyl)phenyl]-5-([(Z)-3-undecenoyl]oxy)pyrimidin,
2-[4-(5-[Propyloxy]-1-pentyl)phenyl]-5-([(Z)-3-dodecenoyl]oxy)pyrimidin,
2-[4-(5-[Propyloxy]-1-pentyl)phenyl]-5-([(E)-4-octenoyl]oxy)pyrimidin, Smp. (C-I) 68°C
2-[4-(5-[Propyloxy]-1-pentyl)phenyl]-5-([(E)-4-nonenoyl]oxy)pyrimidin,
2-[4-(5-[Propyloxy]-1-pentyl)phenyl]-5-([(E)-4-decenoyl]oxy)pyrimidin,
2-[4-(5-[Propyloxy]-1-pentyl)phenyl]-5-([(E)-4-undecenoyl]oxy)pyrimidin,
2-[4-(5-[Propyloxy]-1-pentyl)phenyl]-5-([(E)-4-dodecenoyl]oxy)pyrimidin.
2-[4-(5-[Propyloxy]-1-pentyl)phenyl]-5-([(Z)-5-octenoyl]oxy)pyrimidin,
2-[4-(5-[Propyloxy]-1-pentyl)phenyl]-5-([(Z)-5-nonenoyl]oxy)pyrimidin,
2-[4-(5-[Propyloxy]-1-pentyl)phenyl]-5-([(Z)-5-decenoyl]oxy)pyrimidin,
2-[4-(5-[Propyloxy]-1-pentyl)phenyl]-5-([(Z)-5-undecenoyl]oxy)pyrimidin,
2-[4-(5-[Propyloxy]-1-pentyl)phenyl]-5-([(Z)-5-dodecenoyl]oxy)pyrimidin,
2-[4-(5-[Propyloxy]-1-pentyl)phenyl]-5-([(E)-6-octenoyl]oxy)pyrimidin, Smp. (C-I) 67°C
2-[4-(5-[Propyloxy]-1-pentyl)phenyl]-5-([(E)-6-nonenoyl]oxy)pyrimidin,
2-[4-(5-[Propyloxy]-1-pentyl)phenyl]-5-([(E)-6-decenoyl]oxy)pyrimidin,
2-[4-(5-[Propyloxy]-1-pentyl)phenyl]-5-([(E)-6-undecenoyl]oxy)pyrimidin,
2-[4-(5-[Propyloxy]-1-pentyl)phenyl]-5-([(E)-6-dodecenoyl]oxy)pyrimidin,
2-[4-(5-[Propyloxy]-1-pentyl)phenyl]-5-([7-octenoyl]oxy)pyrimidin, Smp. 54°C,
2-[4-(5-[Propyloxy]-1-pentyl)phenyl]-5-([8-nonenoyl]oxy)pyrimidin,
2-[4-(5-[Propyloxy]-1-pentyl)phenyl]-5-([9-decenoyl]oxy)pyrimidin, Smp. (C-S_{F}) 52°C, S_{F}-S_{C} 57°C, Klp. (S_{C}-I) 61°C,
2-[4-(5-[Propyloxy]-1-pentyl)phenyl]-5-([10-undecenoyl]oxy)pyrimidin,
2-[4-(5-[Propyloxy]-1-pentyl)phenyl]-5-([11-dodecenoyl]oxy)pyrimidin,
2-[4-(5-[Methoxy]-1-pentyl)-4'-biphenyl]-5-([(Z)-3-pentenyl)oxy]pyrimidin,
2-[4-(5-[Methoxy]-1-pentyl)-4'-biphenyl]-5-([(Z)-3-hexenyl)oxy]pyrimidin,
2-[4-(5-[Methoxy]-1-pentyl)-4'-biphenyl]-5-([(Z)-3-heptenyl)oxy]pyrimidin,
2-[4-(5-[Methoxy]-1-pentyl)-4'-biphenyl]-5-([(Z)-3-octenyl)oxy]pyrimidin,

### Beispiel 2

0,14 g Propansäure, 0,8 g (-)-2-[4-(3-Hydroxy-1-butyl)phenyl]-5-[(Z)-3-(octenyl)oxy]pyrimidin, 0,44 g N,N'-Dicyclohexylcarbodiimid, 0,04 g 4-(Dimethylamino)pyridin und 25 ml Dichlormethan wurden in analoger Weise zu Beispiel 1 zu (-)-2-[4-(3-[(Propanoyl)oxy]-1-butyl)phenyl]-5-[(Z)-3-(octenyl)oxy]pyrimidin umgesetzt.

Das als Ausgangsmaterial verwendete (-)-2-[4-(3-Hydroxy-1-butyl)phenyl]-5-[(Z)-3-(octenyl)oxy]pyrimidin wurde wie folgt hergestellt:
a) 12 g 2-(4-Bromphenyl)-5-(äthoxy)pyrimidin, 12 g 2-Acetoxy-3-buten, 10 g N-tert.Butylamin, 20 ml N-Methylpyrrolidon, 0,12 g Palladium(II)-acetat und 0,3 g Triphenylphosphin wurden in analoger Weise zu Beispiel 1(d) zu 9 g 2-[4-(3-Acetoxy-1-butenyl)phenyl]-5-äthoxypyrimidin umgesetzt.
b) 9 g 2-[4-(3-Acetoxy-1-butenyl)phenyl]-5-äthoxypyrimidin, 2 g Palladium auf Aktivkohle (10 wt%) und 150 ml Tetrahydrofuran wurden in analoger Weise zu Beispiel 1(e) zu 8,2 g 2-(3-Acetoxy-1-butyl)phenyl]-5-(äthoxy)pyrimidin hydriert.
c) Ein Gemisch von 250 ml 0,3M Phosphat-Puffer-Lösung (pH 7,0), 25 ml Chloroform und 2,5 g Lipase (Genus Arthobacter) wurde mit 8,2 g 2-[4-(3-Acetoxy-1-butyl)phenyl]-5-(äthoxy)pyrimidin versetzt. Das Reaktionsgemisch wurde bei 36-38°C während 30 Stunden erwärmt, dann dreimal mit je 50 ml Diäthyläther extrahiert. Die vereinigten organischen Phasen wurden zweimal mit je 50 ml konzentrierter Kochsalz-Lösung gewaschen, über Magnesiumsulfat getrocknet, filtriert und anschliessend eingeengt. Chromatographie des Rückstandes an Kieselgel mit Hexan/Aethylacetat (Vol. 7:3) und Umkristallisation aus Aethylalkohol ergab 2,9 g (-)-2-[4-(3-Hydroxy-1-butyl)phenyl]-5-(äthoxy)pyrimidin und 3,1 g reines (-)-2-[4-(3-Acetoxy-1-butyl)phenyl]-5-(äthoxy)pyrimidin.
d) 2,9 g (-)-2-[4-(3-Hydroxy-1-butyl)phenyl]-5-(äthoxy)pyrimidin, 3,1 g (-)-2-[4-(3-Acetexy-1-butyl)phenyl]-5-(äthoxy)pyrimidin, 8 g Natriumhydroxid und 60 ml Diäthylenglykol wurden in analoger Weise zu Beispiel 1(c) zu 4,2 g (-)-2-[4-(3-Hydroxy-1-butyl)phenyl]-5-hydroxypyrimidin umgesetzt.
e) 0,33 g (Z)-3-Octensäure, 1,0 g (-)-2-[4-(3-Hydroxy-1-butyl)phenyl]-5-hydroxypyrimidin, 0,7 g N,N'-Dicyclohexylcarbodiimid, 0,04 g 4-(Dimethylamino)pyridin und 25 ml Dichlormethan wurden in analoger Weise zu Beispiel 1 zu 0,8 g (-)-2-[4-(3-Hydroxy-1-butyl)phenyl]-5-[(Z)-3-(octenyl)oxy]pyrimidin umgesetzt.

In analoger Weise können folgende Verbindungen hergestellt werden:
(-)-2-[4-(3-[(Propanoyl)oxy]-1-butyl)phenyl]-5-([(E)-2-nonenoyl]oxy)pyrimidin,
(-)-2-[4-(3-[(Propanoyl)oxy]-1-butyl)phenyl]-5-([(E)-2-decenoyl]oxy)pyrimidin,
(-)-2-[4-(3-[(Propanoyl)oxy]-1-butyl)phenyl]-5-([(E)-2-undecenoyl]oxy)pyrimidin,
(-)-2-[4-(3-[(Propanoyl)oxy]-1-butyl)phenyl]-5-([(E)-2-dodecenoyl]oxy)pyrimidin,
(-)-2-[4-(3-[(Propanoyl)oxy]-1-butyl)phenyl]-5-([(Z)-3-nonenoyl]oxy)pyrimidin,
(-)-2-[4-(3-[(Propanoyl)oxy]-1-butyl)phenyl]-5-([(Z)-3-decenoyl]oxy)pyrimidin,
(-)-2-[4-(3-[(Propanoyl)oxy]-1-butyl)phenyl]-5-([(Z)-3-undecenoyl]oxy)pyrimidin,
(-)-2-[4-(3-[(Propanoyl)oxy]-1-butyl)phenyl]-5-([(Z)-3-dodecenoyl]oxy)pyrimidin,
(-)-2-[4-(3-[(Propanoyl)oxy]-1-butyl)phenyl]-5-([(E)-4-octenoyl]oxy)pyrimidin,
(-)-2-[4-(3-[(Propanoyl)oxy]-1-butyl)phenyl]-5-([(E)-4-nonenoyl]oxy)pyrimidin,
(-)-2-[4-(3-[(Propanoyl)oxy]-1-butyl)phenyl]-5-([(E)-4-decenoyl]oxy)pyrimidin,
(-)-2-[4-(3-[(Propanoyl)oxy]-1-butyl)phenyl]-5-([(E)-4-undecenoyl]oxy)pyrimidin,
(-)-2-[4-(3-[(Propanoyl)oxy]-1-butyl)phenyl]-5-([(E)-4-dodecenoyl]oxy)pyrimidin,
(-)-2-[4-(3-[(Propanoyl)oxy]-1-butyl)phenyl]-5-([(Z)-5-octenoyl]oxy)pyrimidin,
(-)-2-[4-(3-[(Propanoyl)oxy]-1-butyl)phenyl]-5-([(Z)-5-nonenoyl]oxy)pyrimidin,
(-)-2-[4-(3-[(Propanoyl)oxy]-1-butyl)phenyl]-5-([(Z)-5-decenoyl]oxy)pyrimidin,
(-)-2-[4-(3-[(Propanoyl)oxy]-1-butyl)phenyl]-5-([(Z)-5-undecenoyl]oxy)pyrimidin,
(-)-2-[4-(3-[(Propanoyl)oxy]-1-butyl)phenyl]-5-([(Z)-5-dodecenoyl]oxy)pyrimidin,
(-)-2-[4-(3-[(Propanoyl)oxy]-1-butyl)phenyl]-5-([(E)-6-octenoyl]oxy)pyrimidin,
(-)-2-[4-(3-[(Propanoyl)oxy]-1-butyl)phenyl]-5-([(E)-6-nonenoyl]oxy)pyrimidin,
(-)-2-[4-(3-[(Propanoyl)oxy]-1-butyl)phenyl]-5-([(E)-6-decenoyl]oxy)pyrimidin,
(-)-2-[4-(3-[(Propanoyl)oxy]-1-butyl)phenyl]-5-([(E)-6-undecenoyl]oxy)pyrimidin,
(-)-2-[4-(3-[(Propanoyl)oxy]-1-butyl)phenyl]-5-([(E)-6-dodecenoyl]oxy)pyrimidin,
(-)-2-[4-(3-[(Propanoyl)oxy]-1-butyl)phenyl]-5-([7-octenoyl]oxy)pyrimidin,
(-)-2-[4-(3-[(Propanoyl)oxy]-1-butyl)phenyl]-5-([8-nonenoyl]oxy)pyrimidin,
(-)-2-[4-(3-[(Propanoyl)oxy]-1-butyl)phenyl]-5-([9-decenoyl]oxy)pyrimidin,
(-)-2-[4-(3-[(Propanoyl)oxy]-1-butyl)phenyl]-5-([10-undecenoyl]oxy)pyrimidin,
(-)-2-[4-(3-[(Propanoyl)oxy]-1-butyl)phenyl]-5-([11-dodecenoyl]oxy)pyrimidin,
(-)-2-[4-(5-[(Propanoyl)oxy]-1-hexyl)phenyl]-5-([(E)-2-octenoyl]oxy)pyrimidin,
(-)-2-[4-(5-[(Propanoyl)oxy]-1-hexyl)phenyl]-5-([(E)-2-nonenoyl]oxy)pyrimidin,
(-)-2-[4-(5-[(Propanoyl)oxy]-1-hexyl)phenyl]-5-([(E)-2-decenoyl]oxy)pyrimidin,
(-)-2-[4-(5-[(Propanoyl)oxy]-1-hexyl)phenyl]-5-([(E)-2-undecenoyl]oxy)pyrimidin,
(-)-2-[4-(5-[(Propanoyl)oxy]-1-hexyl)phenyl]-5-([(E)-2-dodecenoyl]oxy)pyrimidin,
(-)-2-[4-(5-[(Propanoyl)oxy]-1-hexyl)phenyl]-5-([(Z)-3-octenoyl]oxy)pyrimidin,
(-)-2-[4-(5-[(Propanoyl)oxy]-1-hexyl)phenyl]-5-([(Z)-3-nonenoyl]oxy)pyrimidin,
(-)-2-[4-(5-[(Propanoyl)oxy]-1-hexyl)phenyl]-5-([(Z)-3-decenoyl]oxy)pyrimidin,
(-)-2-[4-(5-[(Propanoyl)oxy]-1-hexyl)phenyl]-5-([(Z)-3-undecenoyl]oxy)pyrimidin,
(-)-2-[4-(5-[(Propanoyl)oxy]-1-hexyl)phenyl]-5-([(Z)-3-dodecenoyl]oxy)pyrimidin,
(-)-2-[4-(5-[(Propanoyl)oxy]-1-hexyl)phenyl]-5-([7-octenoyl]oxy)pyrimidin,
(-)-2-[4-(5-[(Propanoyl)oxy]-1-hexyl)phenyl]-5-([8-nonenoyl]oxy)pyrimidin,
(-)-2-[4-(5-[(Propanoyl)oxy]-1-hexyl)phenyl]-5-([9-decenoyl]oxy)pyrimidin,
(-)-2-[4-(5-[(Propenoyl)oxy]-1-hexyl)phenyl]-5-([10-undecenoyl]oxy)pyrimidin,
(-)-2-[4-(5-[(Propenoyl)oxy]-1-hexyl)phenyl]-5-([11-dodecenoyl]oxy)pyrimidin,
(-)-2-[4-(3-Acetoxy-1-butyl)phenyl]-5-([(E)-2-octenoyl]oxy)pyrimidin,
(-)-2-[4-(3-Acetoxy-1-butyl)phenyl]-5-([(E)-2-nonenoyl]oxy)pyrimidin,
(-)-2-[4-(3-Acetoxy-1-butyl)phenyl]-5-([(E)-2-decenoyl]oxy)pyrimidin,
(-)-2-[4-(3-Acetoxy-1-butyl)phenyl]-5-([(E)-2-undecenoyl]oxy)pyrimidin,
(-)-2-[4-(3-Acetoxy-1-butyl)phenyl]-5-([(E)-2-dodecenoyl]oxy)pyrimidin,
(-)-2-[4-(3-Acetoxy-1-butyl)phenyl]-5-([(Z)-3-octenoyl]oxy)pyrimidin,
(-)-2-[4-(3-Acetoxy-1-butyl)phenyl]-5-([(Z)-3-nonenoyl]oxy)pyrimidin,
(-)-2-[4-(3-Acetoxy-1-butyl)phenyl]-5-([(Z)-3-decenoyl]oxy)pyrimidin,
(-)-2-[4-(3-Acetoxy-1-butyl)phenyl]-5-([(Z)-3-undecenoyl]oxy)pyrimidin,
(-)-2-[4-(3-Acetoxy-1-butyl)phenyl]-5-([(Z)-3-dodecenoyl]oxy)pyrimidin,
(-)-2-[4-(3-Acetoxy-1-butyl)phenyl]-5-([7-octenoyl]oxy)pyrimidin,
(-)-2-[4-(3-Acetoxy-1-butyl)phenyl]-5-([8-nonenoyl]oxy)pyrimidin,
(-)-2-[4-(3-Acetoxy-1-butyl)phenyl]-5-([9-decenoyl]oxy)pyrimidin,
(-)-2-[4-(3-Acetoxy-1-butyl)phenyl]-5-([10-undecenenoyl]oxy)pyrimidin,
(-)-2-[4-(3-Acetoxy-1-butyl)phenyl]-5-([11-dodecenenoyl]oxy)pyrimidin,
(-)-2-[4-(5-Acetoxy-1-hexyl)phenyl]-5-([(E)-2-octenenoyl]oxy)pyrimidin,
(-)-2-[4-(5-Acetoxy-1-hexyl)phenyl]-5-([(E)-2-nonenoyl]oxy)pyrimidin,
(-)-2-[4-(5-Acetoxy-1-hexyl)phenyl]-5-([(E)-2-decenoyl]oxy)pyrimidin,
(-)-2-[4-(5-Acetoxy-1-hexyl)phenyl]-5-([(E)-2-undecenoyl]oxy)pyrimidin,
(-)-2-[4-(5-Acetoxy-1-hexyl)phenyl]-5-([(E)-2-dodecenoyl]oxy)pyrimidin,
(-)-2-[4-(5-Acetoxy-1-hexyl)phenyl]-5-([(Z)-3-octenoyl]oxy)pyrimidin,
(-)-2-[4-(5-Acetoxy-1-hexyl)phenyl]-5-([(Z)-3-nonenoyl]oxy)pyrimidin,
(-)-2-[4-(5-Acetoxy-1-hexyl)phenyl]-5-([(Z)-3-decenoyl]oxy)pyrimidin,
(-)-2-[4-(5-Acetoxy-1-hexyl)phenyl]-5-([(Z)-3-undecenoyl]oxy)pyrimidin,
(-)-2-[4-(5-Acetoxy-1-hexyl)phenyl]-5-([(Z)-3-dodecenoyl]oxy)pyrimidin,
(-)-2-[4-(5-Acetoxy-1-hexyl)phenyl]-5-([7-octenoyl]oxy)pyrimidin, Smp. (C-I) 54°C, S_{A}-I (44°C),
(-)-2-[4-(5-Acetoxy-1-hexyl)phenyl]-5-([8-nonenoyl]oxy)pyrimidin,
(-)-2-[4-(5-Acetoxy-1-hexyl)phenyl]-5-([9-decenoyl]oxy)pyrimidin,
(-)-2-[4-(5-Acetoxy-1-hexyl)phenyl]-5-([10-undecenoyl]oxy)pyrimidin,
(-)-2-[4-(5-Acetoxy-1-hexyl)phenyl]-5-([11-dodecenoyl]oxy)pyrimidin,
(-)-2-[4-(3-Acetoxy-1-butyl)-4'-biphenyl]-5-([(E)-2-pentenoyl]oxy)pyrimidin,
(-)-2-[4-(3-Acetoxy-1-butyl)-4'-biphenyl]-5-([(E)-2-hexenoyl]oxy)pyrimidin,
(-)-2-[4-(3-Acetoxy-1-butyl)-4'-biphenyl]-5-([(E)-2-hexenoyl]oxy)pyrimidin,
(-)-2-[4-(3-Acetoxy-1-butyl)-4'-biphenyl]-5-([(E)-2-octenoyl]oxy)pyrimidin,
(-)-2-[4-(3-Acetoxy-1-butyl)-4'-biphenyl]-5-([(Z)-3-pentenoyl]oxy)pyrimidin,
(-)-2-[4-(3-Acetoxy-1-butyl)-4'-biphenyl]-5-([(Z)-3-hexenoyl]oxy)pyrimidin,
(-)-2-[4-(3-Acetoxy-1-butyl)-4'-biphenyl]-5-([(Z)-3-heptenoyl]oxy)pyrimidin,
(-)-2-[4-3-Acetoxy-1-butyl)-4'-biphenyl]-5-([(Z)-3-octenoyl]oxy)pyrimidin,
(-)-2-[4-(5-Acetoxy-1-hexyl)-4'-biphenyl]-5-([(E)-2-pentenoyl]oxy)pyrimidin,
(-)-2-[4-(5-Acetoxy-1-hexyl)-4'-biphenyl]-5-([(E)-2-hexenoyl]oxy)pyrimidin,
(-)-2-[4-(5-Acetoxy-1-hexyl)-4'-biphenyl]-5-([(E)-2-heptenoyl]oxy)pyrimidin,
(-)-2-[4-(5-Acetoxy-1-hexyl)-4'-biphenyl]-5-([(E)-2-octenoyl]oxy)pyrimidin,
(-)-2-[4-(5-Acetoxy-1-hexyl)-4'-biphenyl]-5-([(Z)-3-pentenoyl]oxy)pyrimidin,
(-)-2-[4-(5-Acetoxy-1-hexyl)-4'-biphenyl]-5-([(Z)-3-hexenoyl]oxy)pyrimidin,
(-)-2-[4-(5-Acetoxy-1-hexyl)-4'-biphenyl]-5-([(Z)-3-heptenoyl]oxy)pyrimidin,
(-)-2-[4-(5-Acetoxy-1-hexyl)-4'-biphenyl]-5-([(Z)-3-octenoyl]oxy)pyrimidin.

### Beispiel 3

Ein Gemisch von 0,5 g 2-[4-(5-[Propyloxy]-1-pentyl)phenyl]-5-hydroxypyrimidin, 0,3 g (Z)-3-Octen-1-ol, 0,4 g Azodicarbonsäure-diäthylester, 0,6 g Triphenylphosphin und 25 ml absolutem Tetrahydrofuran wurde über Nacht bei Raumtemperatur gerührt und dann eingeengt. Der Rückstand wurde mit 50 ml heissem Hexan aufgeschlämmt und filtriert. Das Filtrat wurde eingeengt. Chromatographie des Rückstandes an Kieselgel mit Toluol/Hexan (Vol. 1:1) und Umkristallisation aus Methanol ergab reinen 2-[4-(5-[Propyloxyl-1-pentyl)phenyl]-5-([(Z)-3-octenyl]oxy)pyrimidin, Smp. (C-I) 38°C.

In analoger Weise können folgende Verbindungen hergestellt werden:
2-[4-(6-[Propyloxy]-1-hexyl)phenyl]-5-([(E)-2-octenyl]oxy)pyrimidin,
2-[4-(6-[Propyloxy]-1-hexyl)phenyl]-5-([(E)-2-nonenyl]oxy)pyrimidin,
2-[4-(6-[Propyloxy]-1-hexyl)phenyl]-5-([(E)-2-decenyl]oxy)pyrimidin,
2-[4-(6-[Propyloxy]-1-hexyl)phenyl]-5-([(E)-2-undecenyl]oxy)pyrimidin,
2-[4-(6-[Propyloxy]-1-hexyl)phenyl]-5-([(E)-2-dodecenyl]oxy)pyrimidin,
2-[4-(6-[Propyloxy]-1-hexyl)phenyl]-5-([(Z)-3-octenyl]oxy)pyrimidin,
2-[4-(6-[Propyloxy]-1-hexyl)phenyl]-5-([(Z)-3-nonenyl]oxy)pyrimidin,
2-[4-(6-[Propyloxy]-1-hexyl)phenyl]-5-([(Z)-3-decenyl]oxy)pyrimidin,
2-[4-(6-[Propyloxy]-1-hexyl)phenyl]-5-([(Z)-3-undecenyl]oxy)pyrimidin,
2-[4-(6-[Propyloxy]-1-hexyl)phenyl]-5-([(Z)-3-dodecenyl]oxy)pyrimidin,
2-[4-(5-[Propyloxy]-1-pentyl)phenyl]-5-([(E)-2-octenyl]oxy)pyrimidin, Smp. (C-S_{C}) 36°C, S_{C}-S_{A} 60°C, Klp, (S_{A}-I) 65°C,
2-[4-(5-[Propyloxy]-1-pentyl)phenyl]-5-([(E)-2-nonenyl]oxy)pyrimidin,
2-[4-(5-[Propyloxy]-1-pentyl)phenyl]-5-([(E)-2-decenyl]oxy)pyrimidin,
2-[4-(5-[Propyloxy]-1-pentyl)phenyl]-5-([(E)-2-undecenyl]oxy)pyrimidin,
2-[4-(5-[Propyloxy]-1-pentyl)phenyl]-5-([(E)-2-dodecenyl]oxy)pyrimidin,
2-[4-(5-[Propyloxy]-1-pentyl)phenyl]-5-([(Z)-3-nonenyl]oxy)pyrimidin,
2-[4-(5-[Propyloxy]-1-pentyl)phenyl]-5-([(Z)-3-decenyl]oxy)pyrimidin,
2-[4-(5-[Propyloxy]-1-pentyl)phenyl]-5-([(Z)-3-undecenyl]oxy)pyrimidin,
2-[4-(5-[Propyloxy]-1-pentyl)phenyl]-5-([(Z)-3-dodecenyl]oxy)pyrimidin,
2-[4-(5-[Propyloxy]-1-pentyl)phenyl]-5-([(E)-4-octenyl]oxy)pyrimidin,
2-[4-(5-[Propyloxy]-1-pentyl)phenyl]-5-([(E)-4-nonenyl]oxy)pyrimidin,
2-[4-(5-[Propyloxy]-1-pentyl)phenyl]-5-([(E)-4-decenyl]oxy)pyrimidin,
2-[4-(5-[Propyloxy]-1-pentyl)phenyl]-5-([(E)-4-undecenyl]oxy)pyrimidin,
2-[4-(5-[Propyloxy]-1-pentyl)phenyl]-5-([(E)-4-dodecenyl]oxy)pyrimidin,
2-[4-(5-[Propyloxy]-1-pentyl)phenyl]-5-([(Z)-5-octenyl]oxy)pyrimidin, S_{C}-S_{A} 33°C, Klp. (S_{A}-I) 37°C,
2-[4-(5-[Propyloxy]-1-pentyl)phenyl]-5-([(Z)-5-nonenyl]oxy)pyrimidin,
2-[4-(5-[Propyloxy]-1-pentyl)phenyl]-5-([(Z)-5-decenyl]oxy)pyrimidin,
2-[4-(5-[Propyloxy]-1-pentyl)phenyl]-5-([(Z)-5-undecenyl]oxy)pyrimidin,
2-[4-(5-[Propyloxy]-1-pentyl)phenyl]-5-([(Z)-5-dodecenyl]oxy)pyrimidin,
2-[4-(5-[Propyloxy]-1-pentyl)phenyl]-5-([(E)-6-octenyl]oxy)pyrimidin,
2-[4-(5-[Propyloxy]-1-pentyl)phenyl]-5-([(E)-6-nonenyl]oxy)pyrimidin,
2-[4-(5-[Propyloxy]-1-pentyl)phenyl]-5-([(E)-6-decenyl]oxy)pyrimidin,
2-[4-(5-[Propyloxy]-1-pentyl)phenyl]-5-([(E)-6-undecenyl]oxy)pyrimidin,
2-[4-(5-[Propyloxy]-1-pentyl)phenyl]-5-([(E)-6-dodecenyl]oxy)pyrimidin,
2-[4-(5-[Propyloxy]-1-pentyl)phenyl]-5-([7-octenyl]oxy)pyrimidin, Smp. (C-S_{C}) 15°C, S_{C}-S_{A} 35°C, Klp. (S_{A}-I) 58°C,
2-[4-(5-[Propyloxy]-1-pentyl)phenyl]-5-([8-nonenyl]oxy)pyrimidin,
2-[4-(5-[Propyloxy]-1-pentyl)phenyl]-5-([9-decenyl]oxy)pyrimidin,
2-[4-(5-[Propyloxy]-1-pentyl)phenyl]-5-([10-undecenyl]oxy)pyrimidin,
2-[4-(5-[Propyloxy]-1-pentyl)phenyl]-5-([11-dodecenyl]oxy)pyrimidin, Smp. (C-S) 19°C, S₃-S₂ 36°C, S₂-S_{C} 59°C, Klp. (S_{C}-I) 70°C,
2-[4-(5-[Butyloxy]-1-pentyl)phenyl]-5-([(E)-2-octenyl]oxy)pyrimidin, Smp. (C-S_{C}) 31°C, S_{C}-S_{A} 56°C, Klp. (S_{A}-I) 61°C,
2-[4-(5-Methoxy-1-pentyl)-4'-biphenyl]-5-([(E)-2-pentenyl]oxy)pyrimidin,
2-[4-(5-Methoxy-1-pentyl)-4'-biphenyl]-5-([(E)-2-hexenyl]oxy)pyrimidin,
2-[4-(5-Methoxy-1-pentyl)-4'-biphenyl]-5-([(E)-2-heptenyl]oxy)pyrimidin,
2-[4-(5-Methoxy-1-pentyl)-4'-biphenyl]-5-([(E)-2-octenyl]oxy)pyrimidin,
2-[4-(5-Methoxy-1-pentyl)-4'-biphenyl]-5-([(Z)-3-pentenyl]oxy)pyrimidin,
2-[4-(5-Methoxy-1-pentyl)-4'-biphenyl]-5-([(Z)-3-hexenyl]oxy)pyrimidin,
2-[4-(5-Methoxy-1-pentyl)-4'-biphenyl]-5-([(Z)-3-heptenyl]oxy)pyrimidin,
2-[4-(5-Methoxy-1-pentyl)-4'-biphenyl]-5-([(Z)-3-octenyl]oxy)pyrimidin.

### Beispiel 4

0,5 g (Z)-3-Octensäure, 1,0 g 2-[4-(5-[Propyloxy]-1-pentyl)phenyl]-5-hydroxypyridin, 0,7 g N,N'-Dicyclohexylcarbodiimid, 0,04 g (Dimethylamino)pyridin und 50 ml Dichlormethan wurden in analoger Weise zu Beispiel 1 zu 2-[4-(5-[Propyloxy]-1-pentyl)phenyl]-5-([(Z)-3-octenoyl)oxy)pyridin umgesetzt.

Das als Ausgangsmaterial verwendete 2-[5-(5-[Propyloxy]-1-pentyl)phenyl]-5-hydroxypyridin wurde wie folgt hergestellt:
a) Eine aus 0,8 g Magnesium, 8,0 g 1,4-Dibrombenzol und 50 ml Tetrahydrofuran bereitete Grignard-Reagens-Lösung wurde bei 0-5°C innert 30 Minuten in ein Gemisch aus 6,6 g 5-Trimethylsilyloxy-2-chlorpyridin, 75 ml Tetrahydrofuran und 0,4 g 1,3-Bis(diphenylphosphino)propannickel-(II)-chlorid getropft. Das Reaktionsgemisch wurde 3 Stunden gerührt, über Nacht stehen gelassen, dann mit wässriger Natriumhydrogencarbonat-Lösung auf pH 8 gestellt und mit Diäthyläther verdünnt. Die organische Phase wurde mit Wasser neutral gewaschen, über Natriumsulfat getrocknet und eingeengt. Chromatographische Reinigung des Rückstandes an Kieselgel mit Hexan/Aethylacetat (Vol. 4:1) lieferte 5,5 g 5-Hydroxy-2-(4-bromphenyl)pyridin.
b) Ein Gemisch von 5,5 g 5-Hydroxy-2-(4-bromphenyl)pyridin, 2,4 g Triäthylamin und 50 ml Toluol wurde bei 0°C und unter Stickstoffbegasung vorgelegt und dann tropfenweise mit 3,1 g Trimethylchlorsilan versetzt. Das Reaktionsgemisch wurde über Nacht bei Raumtemperatur gerührt, dann mit 50 ml gesättigter Natriumbicarbonat-Lösung versetzt und dreimal mit je 100 ml Diäthyläther extrahiert. Die vereinigten organischen Phasen wurden zweimal mit gesättigter Natriumbicarbonat-Lösung gewaschen, über Magnesiumsulfat getrocknet, filtriert und anschliessend eingeengt. Dies ergab 6,0 g 5-Trimethylsilyloxy-2-(4-bromphenyl)pyridin.
c) 6,0 g 5-Trimethylsilyloxy-2-(4-bromphenyl)pyridin, 2,5 g 4-Penten-1-ol, 4 g Natriumbicarbonat-Lösung, 20 ml N-Methyl-pyrrolidon, 0,03 g Palladium(II)-acetat und 0,11 g Triphenylphosphin wurden in analoger Weise zu Beispiel 1(b) zu 5,4 g 5-Trimethylsilyloxy-2-(5-[5-hydroxy-1-pentenyl]phenyl)pyridin umgesetzt.
d) 5,4 g 5-Trimethylsilyloxy-2-(5-[5-hydroxy-1-pentenyl]phenyl)pyridin, 1 g Palladium auf Aktivkohle (10 w/w%) und 50 ml Aethylacetat wurden in analoger Weise zu Beispiel 1(c) zu 5,1 g 5-Trimethylsilyloxy-2-(5-[5-hydroxy-1-pentyl]phenyl)pyridin umgesetzt.
e) 5,1 g 5-Trimethylsilyloxy-2-(5-[5-hydroxy-1-pentyl]phenyl)pyridin, 0,7 g Natriumhydrid (60-65 wt%), 2,5 g 1-Brompropan und 100 ml Tetrahydrofuran wurden analog zu Beispiel 1(f) zu 3,6 g 5-Hydroxy-2-(5-[5-(propyloxy)-1-pentyl]phenyl)pyridin umgesetzt.

In analoger Weise können folgende Verbindungen hergestellt werden:
2-[4-(5-[Propyloxy]-1-pentyl)phenyl]-5-([(E)-2-octenoyl]oxy)pyridin,
2-[4-(5-[Propyloxy]-1-pentyl)phenyl]-5-([(E)-2-nonenoyl]oxy)pyridin,
2-[4-(5-[Propyloxy]-1-pentyl)phenyl]-5-([(E)-2-decenoyl]oxy)pyridin,
2-[4-(5-[Propyloxy]-1-pentyl)phenyl]-5-([(E)-2-undecenoyl]oxy)pyridin,
2-[4-(5-[Propyloxy]-1-pentyl)phenyl]-5-([(E)-2-dodecenoyl]oxy)pyridin,
2-[4-(5-[Propyloxy]-1-pentyl)phenyl]-5-([(Z)-3-nonenoyl]oxy)pyridin,
2-[4-(5-[Propyloxy]-1-pentyl)phenyl]-5-([(Z)-3-decenoyl]oxy)pyridin,
2-[4-(5-[Propyloxy]-1-pentyl)phenyl]-5-([(Z)-3-undecenoyl]oxy)pyridin,
2-[4-(5-[Propyloxy]-1-pentyl)phenyl]-5-([(Z)-3-dodecenoyl]oxy)pyridin,
2-[4-(5-[Propyloxy]-1-pentyl)phenyl]-5-([(E)-4-octenoyl]oxy)pyridin,
2-[4-(5-[Propyloxy]-1-pentyl)phenyl]-5-([(E)-4-nonenoyl]oxy)pyridin,
2-[4-(5-[Propyloxy]-1-pentyl)phenyl]-5-([(E)-4-decenoyl]oxy)pyridin,
2-[4-(5-[Propyloxy]-1-pentyl)phenyl]-5-([(E)-4-undecenoyl]oxy)pyridin,
2-[4-(5-[Propyloxy]-1-pentyl)phenyl]-5-([(E)-4-dodecenoyl]oxy)pyridin,
2-[4-(5-[Propyloxy]-1-pentyl)phenyl]-5-([(Z)-5-octenoyl]oxy)pyridin,
2-[4-(5-[Propyloxy]-1-pentyl)phenyl]-5-([(Z)-5-nonenoyl]oxy)pyridin,
2-[4-(5-[Propyloxy]-1-pentyl)phenyl]-5-([(Z)-5-decenoyl]oxy)pyridin,
2-[4-(5-[Propyloxy]-1-pentyl)phenyl]-5-([(Z)-5-undecenoyl]oxy)pyridin,
2-[4-(5-[Propyloxy]-1-pentyl)phenyl]-5-([(Z)-5-dodecenoyl]oxy)pyridin,
2-[4-(5-[Propyloxy]-1-pentyl)phenyl]-5-([(E)-6-octenoyl]oxy)pyridin,
2-[4-(5-[Propyloxy]-1-pentyl)phenyl]-5-([(E)-6-nonenoyl]oxy)pyridin,
2-[4-(5-[Propyloxy]-1-pentyl)phenyl]-5-([(E)-6-decenoyl]oxy)pyridin,
2-[4-(5-[Propyloxy]-1-pentyl)phenyl]-5-([(E)-6-undecenoyl]oxy)pyridin,
2-[4-(5-[Propyloxy]-1-pentyl)phenyl]-5-([(E)-6-dodecenoyl]oxy)pyridin,
2-[4-(5-[Propyloxy]-1-pentyl)phenyl]-5-([7-octenoyl]oxy)pyridin, Smp. (C-I) 54°C;
2-[4-(5-[Propyloxy]-1-pentyl)phenyl]-5-([8-nonenoyl]oxy)pyridin,
2-[4-(5-[Propyloxy]-1-pentyl)phenyl]-5-([9-decenoyl]oxy)pyridin,
2-[4-(5-[Propyloxy]-1-pentyl)phenyl]-5-([10-undecenoyl]oxy)pyridin,
2-[4-(5-[Propyloxy]-1-pentyl)phenyl]-5-([11-dodecenoyl]oxy)pyridin,
2-[4-(5-[Methoxy]-1-pentyl)-4'-biphenyl]-5-([(Z)-3-pentenoyl]oxy)pyridin,
2-[4-(5-[Methoxy]-1-pentyl)-4'-biphenyl]-5-([(Z)-3-hexenoyl]oxy)pyridin,
2-[4-(5-[Methoxy]-1-pentyl)-4'-biphenyl]-5-([(Z)-3-heptenoyl]oxy)pyridin,
2-[4-(5-[Methoxy]-1-pentyl)-4'-biphenyl]-5-([(Z)-3-octenoyl]oxy)pyridin.

### Beispiel 5

0,14 g Propansäure, 0,8 g (-)-2-[4-(3-Hydroxy-1-butyl)phenyl]-5-[(Z)-3-(octenyl)oxy]pyridin, 0,44 g N,N'-Dicyclohexylcarbodiimid, 0,04 g 4-(Dimethylamino)pyridin und 50 ml Dichlormethan wurden in analoger Weise zu Beispiel 1 zu 0,7 g (-)-2-[4-(3-[(Propanoyl)oxy]-1-butyl)phenyl]-5-[(Z)-3-(octenoyl)oxy]pyridin umgesetzt.

Das als Ausgangsmaterial verwendete (-)-2-[4-(3-Hydroxy-1-butyl)phenyl]-5-[(Z)-3-(octenoyl)oxy]pyridin wurde wie folgt hergestellt:
a) 17 g 5-Trimethylsilyloxy-2-(4-bromphenyl)pyridin 12 g 2-Acetoxy-3-buten, 10 g N-tert.-Butylamin, 20 ml N-Methyl-pyrrolidon, 0,12 g Palladium-(II)-acetat und 0,3 g Triphenylphosphin wurden in analoger Weise zu Beispiel 1(d) zu 14 g 2-[4-(3-Acetoxy-1-butenyl)phenyl]-5-trimethylsilyloxypyridin umgesetzt.
b) 14 g 2-[4-(3-Acetoxy-1-butenyl)phenyl]-5-trimethylsilyloxy-pyridin, 2 g Palladium auf Aktivkohle (10 wt%) und 200 ml Tetrahydrofuran wurden in analoger Weise zu Beispiel 1(a) zu 12 g 2-[4-(3-Acetoxy-1-butyl)phenyl]-5-trimethylsilyloxy-pyridin hydriert.
c) 12 g 2-[4-(3-Acetoxy-1-butyl)phenyl]-5-trimethylsilyloxy-pyridin, 300 ml 0,3M Phosphat-Puffer-Lösung (pH 7,0), 30 ml Chloroform und 3,0 g Lipase (Genus Arthobacter) wurden zu 4,5 g (-)-2-[4-(3-Hydroxy-1-butyl)phenyl]-5-hydroxy-pyridin und 4,2 g (-)-2-[4-(3-Acetoxy-1-butyl)phenyl]-5-hydroxy-pyridin umgesetzt.
d) 0,35 g (Z)-3-Octensäure, 1,0 g (-)-2-[4-(3-Hydroxy-1-butyl)phenyl]-5-hydroxy-pyridin, 0,7 g N,N'-Dicyclohexylcarbodiimid, 0,04 g 4-(Dimethylamino)pyridin und 25 ml Dichlormethan wurden in analoger Weise zu Beispiel 1 zu 0,75 g (-)-2-[4-(3-Hydroxy-1-butyl)phenyl]-5-[(Z)-3-(octenoyl)oxy]pyridin umgesetzt.

In analoger Weise können folgende Verbindungen hergestellt werden:
(-)-2-[4-(3-[(Propanoyloxy]-1-butyl)phenyl]-5-([(E)-2-octenoyl]oxypyridin,
(-)-2-[4-(3-[(Propanoyl)oxy]-1-butyl)phenyl]-5-([(E)-2-nonenoyl]oxy)pyridin,
(-)-2-[4-(3-[(Propanoyl)oxy]-1-butyl)phenyl]-5-([(E)-2-decenoyl]oxy)pyridin,
(-)-2-[4-(3-[(Propanoyl)oxy]-1-butyl)phenyl]-5-([(E)-2-undecenoyl]oxy)pyridin,
(-)-2-[4-(3-[(Propanoyl)oxy]-1-butyl)phenyl]-5-([(Z)-2-dodecenoyl]oxy)pyridin,
(-)-2-[4-(3-[(Propanoyl)oxy]-1-butyl)phenyl]-5-([(Z)-3-nonenoyl]oxy)pyridin,
(-)-2-[4-(3-[(Propanoyl)oxy]-1-butyl)phenyl]-5-([(Z)-3-decenoyl]oxy)pyridin,
(-)-2-[4-(3-[(Propanoyl)oxy]-1-butyl)phenyl]-5-([(Z)-3-undecenoyl]oxy)pyridin,
(-)-2-[4-(3-[(Propanoyl)oxy]-1-butyl)phenyl]-5-([(Z)-3-dodecenoyl]oxy)pyridin,
(-)-2-[4-(3-[(Propanoyl)oxy]-1-butyl)phenyl]-5-([(E)-4-octenoyl]oxy)pyridin,
(-)-2-[4-(3-[(Propanoyl)oxy]-1-butyl)phenyl]-5-([(E)-4-nonenoyl]oxy)pyridin,
(-)-2-[4-(3-[(Propanoyl)oxy]-1-butyl)phenyl]-5-([(E)-4-decenoyl]oxy)pyridin,
(-)-2-[4-(3-[(Propanoyl)oxy]-1-butyl)phenyl]-5-([(E)-4-undecenoyl]oxy)pyridin,
(-)-2-[4-(3-[(Propanoyl)oxy]-1-butyl)phenyl]-5-([(E)-4-dodecenoyl]oxy)pyridin,
(-)-2-[4-(3-[(Propanoyl)oxy]-1-butyl)phenyl]-5-([(Z)-5-octenoyl]oxy)pyridin,
(-)-2-[4-(3-[(Propanoyl)oxy]-1-butyl)phenyl]-5-([(Z)-5-nonenoyl]oxy)pyridin,
(-)-2-[4-(3-[(Propanoyl)oxy]-1-butyl)phenyl]-5-([(Z)-5-decenoyl]oxy)pyridin,
(-)-2-[4-(3-[(Propanoyl)oxy]-1-butyl)phenyl]-5-([(Z)-5-undecenoyl]oxy)pyridin,
(-)-2-[4-(3-[(Propanoyl)oxy]-1-butyl)phenyl]-5-([(Z)-5-dodecenoyl]oxy)pyridin,
(-)-2-[4-(3-[(Propanoyl)oxy]-1-butyl)phenyl]-5-([(E)-6-octenoyl]oxy)pyridin,
(-)-2-[4-(3-[(Propanoyl)oxy]-1-butyl)phenyl]-5-([(E)-6-nonenoyl]oxy)pyridin,
(-)-2-[4-(3-[(Propanoyl)oxy]-1-butyl)phenyl]-5-([(E)-6-decenoyl]oxy)pyridin,
(-)-2-[4-(3-[(Propanoyl)oxy]-1-butyl)phenyl]-5-([(E)-6-undecenoyl]oxy)pyridin,
(-)-2-[4-(3-[(Propanoyl)oxy]-1-butyl)phenyl]-5-([(E)-6-dodecenoyl]oxy)pyridin,
(-)-2-[4-(3-[(Propanoyl)oxy]-1-butyl)phenyl]-5-([7-octenoyl]oxy)pyridin,
(-)-2-[4-(3-[(Propanoyl)oxy]-1-butyl)phenyl]-5-([8-nonenoyl]oxy)pyridin,
(-)-2-[4-(3-[(Propanoyl)oxy]-1-butyl)phenyl]-5-([9-decenoyl]oxy)pyridin,
(-)-2-[4-(3-[(Propanoyl)oxy]-1-butyl)phenyl]-5-([10-undecenoyl]oxy)pyridin,
(-)-2-[4-(3-[(Propanoyl)oxy]-1-butyl)phenyl]-5-([11-dodecenoyl]oxy)pyridin,
(-)-2-[4-(3-Acetoxy-1-butyl)-4'-biphenyl]-5-([(Z)-3-pentenoyl]oxy)pyridin,
(-)-2-[4-(3-Acetoxy-1-butyl)-4'-biphenyl]-5-([(Z)-3-hexenoyl]oxy)pyridin,
(-)-2-[4-(3-Acetoxy-1-butyl)-4'-biphenyl]-5-([(Z)-3-heptenoyl]oxy)pyridin,
(-)-2-[4-(3-Acetoxy-1-butyl)-4'-biphenyl]-5-([(Z)-3-octenoyl]oxy)pyridin.

### Beispiel 6

1,0 g 2-[4-(5-[Propyloxy]-1-pentyl)phenyl]-5-hydroxypyridin, 0,42 g (Z-3-Octen-1-ol, 0,58 g Azodicarbonsäure-diäthylester, 0,88 g Triphenylphosphin und 100 ml Tetrahydrofuran wurden in analoger Weise zu Beispiel 3 zu 0,8 g 2-[4-(5-[Propyloxy]-1-pentyl)phenyl]-5-([(Z)-3-octenyl]oxy)pyridin umgesetzt.

In analoger Weise können folgende Verbindungen hergestellt werden:
2-[4-(5-[Propyloxy]-1-pentyl)phenyl]-5-([(Z)-3-octenyl]oxypyridin,
2-[4-(5-[Propyloxy]-1-pentyl)phenyl]-5-([(E)-2-nonenyl]oxy)pyridin,
2-[4-(5-[Propyloxy]-1-pentyl)phenyl]-5-([(E)-2-decenyl]oxy)pyridin,
2-[4-(5-[Propyloxy]-1-pentyl)phenyl]-5-([(E)-2-undecenyl]oxy)pyridin,
2-[4-(5-[Propyloxy]-1-pentyl)phenyl]-5-([(Z)-2-dodecenyl]oxy)pyridin,
2-[4-(5-[Propyloxy]-1-pentyl)phenyl]-5-([(Z)-3-nonenyl]oxy)pyridin,
2-[4-(5-[Propyloxy]-1-pentyl)phenyl]-5-([(Z)-3-decenyl]oxy)pyridin,
2-[4-(5-[Propyloxy]-1-pentyl)phenyl]-5-([(Z)-3-undecenyl]oxy)pyridin,
2-[4-(5-[Propyloxy]-1-pentyl)phenyl]-5-([(Z)-3-dodecenyl]oxy)pyridin,
2-[4-(5-[Propyloxy]-1-pentyl)phenyl]-5-([(E)-4-octenyl]oxy)pyridin,
2-[4-(5-[Propyloxy]-1-pentyl)phenyl]-5-([(E)-4-nonenyl]oxy)pyridin,
2-[4-(5-[Propyloxy]-1-pentyl)phenyl]-5-([(E)-4-decenyl]oxy)pyridin,
2-[4-(5-[Propyloxy]-1-pentyl)phenyl]-5-([(E)-4-undecenyl]oxy)pyridin,
2-[4-(5-[Propyloxy]-1-pentyl)phenyl]-5-([(E)-4-dodecenyl]oxy)pyridin,
2-[4-(5-[Propyloxy]-1-pentyl)phenyl]-5-([(Z)-5-octenyl]oxy)pyridin,
2-[4-(5-[Propyloxy]-1-pentyl)phenyl]-5-([(Z)-5-nonenyl]oxy)pyridin,
2-[4-(5-[Propyloxy]-1-pentyl)phenyl]-5-([(Z)-5-decenyl]oxy)pyridin,
2-[4-(5-[Propyloxy]-1-pentyl)phenyl]-5-([(Z)-5-undecenyl]oxy)pyridin,
2-[4-(5-[Propyloxy]-1-pentyl)phenyl]-5-([(Z)-5-dodecenyl]oxy)pyridin,
2-[4-(5-[Propyloxy]-1-pentyl)phenyl]-5-([(E)-6-octenyl]oxy]pyridin,
2-[4-(5-[Propyloxy]-1-pentyl)phenyl]-5-([(E)-6-nonenyl]oxy]pyridin,
2-[4-(5-[Propyloxy]-1-pentyl)phenyl]-5-([(E)-6-decenyl]oxy]pyridin,
2-[4-(5-[Propyloxy]-1-pentyl)phenyl]-5-([(E)-6-undecenyl]oxy]pyridin,
2-[4-(5-[Propyloxy]-1-pentyl)phenyl]-5-([(E)-6-dodecenyl]oxy]pyridin,
2-[4-(5-[Propyloxy]-1-pentyl)phenyl]-5-([7-octenyl]oxy]pyridin,
2-[4-(5-[Propyloxy]-1-pentyl)phenyl]-5-([8-nonenyl]oxy]pyridin,
2-[4-(5-[Propyloxy]-1-pentyl)phenyl]-5-([9-decenyl]oxy]pyridin,
2-[4-(5-[Propyloxy]-1-pentyl)phenyl]-5-([10-undecenyl]oxy]pyridin,
2-[4-(5-[Propyloxy]-1-pentyl)phenyl]-5-([11-dodecenyl]oxy]pyridin,
2-[4-(5-[Methoxy]-1-pentyl)-4'-biphenyl]-5-([(E)-2-pentenyl]oxy)pyridin,
2-[4-(5-[Methoxy]-1-pentyl)-4'-biphenyl]-5-([(E)-2-hexenyl]oxy)pyridin,
2-[4-(5-[Methoxy]-1-pentyl)-4'-biphenyl]-5-([(E)-2-heptenyl]oxy)pyridin,
2-[4-(5-[Methoxy]-1-pentyl)-4'-biphenyl]-5-([(E)-2-octenyl]oxy)pyridin,
2-[4-(5-[Methoxy]-1-pentyl)-4'-biphenyl]-5-([(Z)-3-pentenyl]oxy)pyridin,
2-[4-(5-[Methoxy]-1-pentyl)-4'-biphenyl]-5-([(Z)-3-hexenyl]oxy)pyridin,
2-[4-(5-[Methoxy]-1-pentyl)-4'-biphenyl]-5-([(Z)-3-heptenyl]oxy)pyridin,
2-[4-(5-[Methoxy]-1-pentyl)-4'-biphenyl]-5-([(Z)-3-octenyl]oxy)pyridin.

## Patentansprüche

1. Verbindungen der allgemeinen Formel 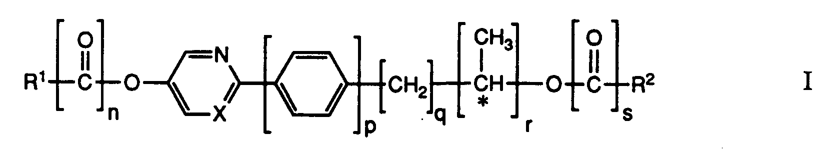 worin
R¹ Alkyl oder Alkenyl mit 5 bis 12 Kohlenstoffatomen bedeutet;
R² Alkyl oder Alkenyl mit 1 bis 9 bzw. 2 bis 9 Kohlenstoffatomen bedeutet; mit der Massgabe, dass mindestens eine der Gruppen R¹ oder R² Alkenyl bedeutet;
X eine CH-Gruppe oder Stickstoff bedeutet;
n entweder 0 oder 1 ist;
q eine ganze Zahl von 2 bis 6 ist;
r entweder 0 oder 1 ist, mit der Massgabe, dass Σ q+r mindestens 3 ist;
s entweder 0 oder 1 ist;
p 1 oder 2 bedeutet, mit der Massgabe, dass wenn p = 2 ist, n für die Zahl 0 steht.

2. Verbindungen gemäss der allgemeinen Formel 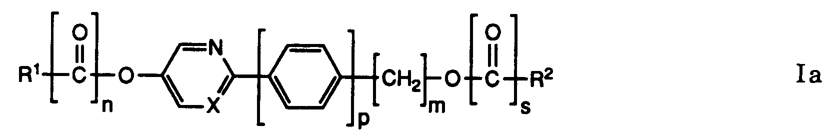 worin m eine ganze Zahl von 3 bis 6 ist, und R¹, n, p, s und R² die in Anspruch 1 angegebenen Bedeutungen haben.

3. Verbindungen gemäss einem der Ansprüche 1 oder 2, dadurch gekennzeichnet, dass R¹ Alkenyl mit 5 bis 10 Kohlenstoffatomen bedeutet; R² Alkyl mit 1 bis 7 Kohlenstoffatomen bedeutet; und X Stickstoff bedeutet.

4. Verbindungen gemäss einem der Ansprüche 1 bis 3 der allgemeinen Formel 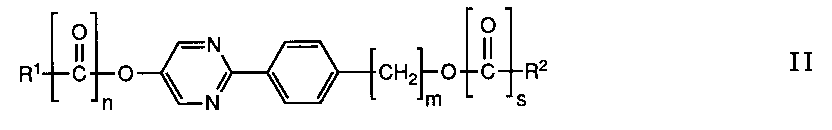 worin
R¹ Alkenyl mit 8 bis 10 Kohlenstoffatomen bedeutet;
R² Alkyl mit 1 bis 5 Kohlenstoffatomen bedeutet;
n entweder 0 oder 1 ist;
m eine ganze Zahl von 3 bis 6 ist;
s entweder 0 oder 1 ist.

5. Flüssigkristallines Gemisch mit mindestens 2 Komponenten, dadurch gekennzeichnet, dass mindestens eine Komponente eine Verbindung der **in Anspruch 1 definierten** allgemeinen Formel I ist.

6. Verwendung der Verbindungen der in Anspruch 1 definierten Formel I für elektro-optische Zwecke.

## Claims

1. Compounds of the general formula 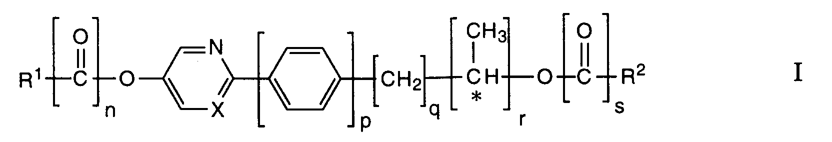 wherein
R¹ signifies alkyl or alkenyl with 5 to 12 carbon atoms;
R² signifies alkyl or alkenyl with 1 to 9 and, respectively, 2 to 9 carbon atoms; with the proviso that at least one of groups R¹ or R² signifies alkenyl;
X signifies a CH group or nitrogen;
n is either 0 or 1;
q is a whole number of 2 to 6;
r is either 0 or 1 with the proviso that Σ q+r is at least 3,
s is either 0 or 1; and
p signifies 1 or 2, with the proviso that n stands for the number 0 when p = 2.

2. Compounds according to the general formula 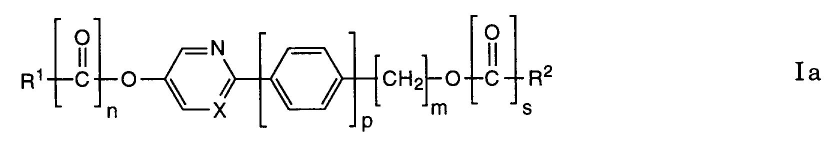 wherein m is a whole number of 3 to 6 and R¹, n, , p, s and R² have the significances given in claim 1.

3. Compounds according to one of claims 1 or 2, characterized in that R¹ signifies alkenyl with 5 to 10 carbon atoms; R² signifies alkyl with 1 to 7 carbon atoms; and X signifies nitrogen.

4. Compounds according to one of claims 1 to 3 of the general formula 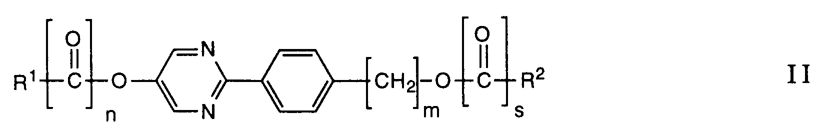 wherein
R¹ signifies alkenyl with 8 to 10 carbon atoms;
R² signifies alkyl with 1 to 5 carbon atoms;
n is either 0 or 1;
m is a whole number of 3 to 6; and
s is either 0 or 1.

5. A liquid crystalline mixture with at least 2 components, characterized in that at least one component is a compound of the general formula I defined in claim 1.

6. The use of the compounds of formula I defined in claim 1 for electro-optical purposes.

## Revendications

1. Composés de formule générale 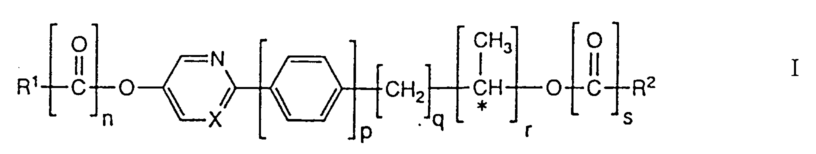 dans laquelle
R¹ représente un groupe alkyle ou alcényle en C5-C12 ;
R² représente un groupe alkyle en C1-C9 ou alcényle en C2-C9, sous réserve que l'un au moins des groupes R¹ et R² représente un groupe alcényle ;
X représente un groupe CH ou l'azote ;
n est égal à 0 ou 1;
q est un nombre entier allant de 2 à 6 ;
r est égal à 0 ou 1, sous réserve que la somme q + r est égale au moins à 3 ;
s est égal à 0 ou 1 ;
p est égal à 1 ou 2, sous réserve que, lorsque p est égal à 2, n est égal à 0.

2. Composés de formule générale 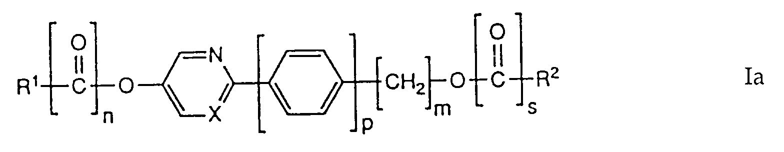 dans laquelle m est un nombre entier allant de 3 à 6, et R¹, n, p, s, et R² ont les significations indiquées dans la revendication 1.

3. Composés selon une des revendications 1 ou 2, caractérisés en ce que R¹ représente un groupe alcényle en C5-C10, R² un groupe alkyle en C1-C7 et X l'azote.

4. Composés selon l'une des revendications 1 à 3, répondant à la formule générale 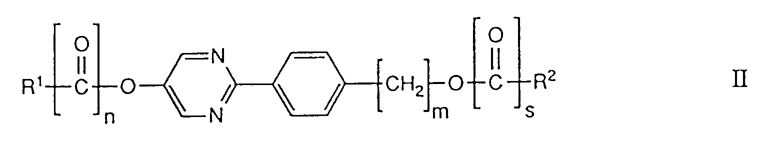 dans laquelle
R¹ représente un groupe alcényle en C8-C10,
R² représente un groupe alkyle en C1-C5,
n est égal à 0 ou 1,
m est un nombre entier allant de 3 à 6 et
s est égal à 0 ou 1.

5. Mélanges à cristaux liquides à au moins deux composants, caractérisé en ce que l'un au moins des composants est un composé de formule générale I définie dans la revendication 1.

6. Utilisation des composés de formule I définie dans la revendication 1 dans des applications électro-optiques.
